# EUROPÄISCHE PATENTSCHRIFT

(11) **EP 2 926 788 B1**
(45) Veröffentlichungstag und Bekanntmachung des Hinweises auf die Patenterteilung: **06.02.2019**
(21) Anmeldenummer: 15160950.0
(22) Anmeldetag: 26.03.2015
(51) Int. Cl.: A61F 13/511, A61L 15/22, D04H 1/4391

(54) **Deckblatt**
Cover sheet
Feuille de garde

(30) Priorität: 04.04.2014 DE 102014004884
(43) Veröffentlichungstag der Anmeldung: 07.10.2015
(73) Patentinhaber: Sandler AG, 95126 Schwarzenbach/Saale (DE)
(72) Erfinder:

(56) Entgegenhaltungen:
- DE-T2- 69 230 246
- US-A1- 2005 227 563

## Beschreibung

Prinzipiell haben persönliche Hygieneprodukte folgenden, schematischen Aufbau:
- Deckblatt als zum Körper hingewandte Lage
- Aufnahme- und Verteillage.
- Speicherlage
- Rückenblatt als flüssigkeitsdichte Außenlage

Das Deckblatt hat dabei die Aufgabe dem Benutzer ein trockenes, weiches, und angenehmes Tragegefühl zu vermitteln, sowie auftreffende Flüssigkeit schnell anzusaugen und an die nachfolgende Aufnahme und Verteillage weiterzuleiten, sowie die Rücknässung zu minimieren. Die Aufnahme und Verteillage dient zur raschen Aufnahme der Flüssigkeit aus dem Deckblatt und gewährleistet die Verteilung der Flüssigkeit über die gesamte Fläche eines Hygieneproduktes. Letztendlich wird die Flüssigkeit in der Speicherschicht immobilisiert.

Einfache Aufbauten von sogenannten Personal Care Produkten (z. B. Damenbinden, Babywindeln und Inkontinenzprodukte) verzichten zum Teil auch auf den Einsatz einer Aufnahme- und Verteillage, sodass dem Deckblatt direkt die Speicherlage folgt. Dies hat allerdings den Nachteil, dass die Speicherlage nicht zu 100% ausgenützt wird und es daher zu partieller Überladung des Produkts und Gelblocking kommt, was sich als erhöhte Rücknässung bemerkbar macht. In diesem Bereich dominieren bezüglich der Weichheit heißluftverfestigte Vliese. Gefertigt werden diese sogenannten Airthrough-Vliese in aller Regel aus 100% PP/PE oder PET/PE Bikokompentenfasern. Gattungsbildend ist hier die EP070163. Die Flüssigkeitsansaugung derartiger Produkte ist allerdings durch die Vielzahl von Verbindungsstellen innerhalb der Vliesstruktur nur bedingt gegeben, des Weiteren wird ein Anteil der Flüssigkeit im Deckblatt zurückgehalten.

Wird zweilagig gearbeitet, d.h. mit einer Kombination aus Deckblatt und nachgeschalteter Aufnahme- und Verteillage, so kommen für dem Stand der Technik entsprechende Deckblätter thermobondierte Spinnvliese oder Stapelfaservliese, heißluftverfestigte Vliese oder perforierten Versionen aus diesen genannten in Frage. Weiterhin findet man perforierte Foliensysteme im Markt.

Die dem Deckblatt dann nachgeschaltete Aufnahme- und Verteillage hat die Aufgabe zum Einen, die Flüssigkeit zwischen zu speichern, um einem Gelblocking in der Speicherlage vorzubeugen, zum anderen muss die Flüssigkeit aber auch möglichst über die ganze Fläche des Hygieneartikels verteilt werden, sodass die Speicherlage vollständig genutzt wird.

Das Zusammenspiel dieser beiden Lagen, Decklage sowie Aufnahme- und Verteillage ist entscheidet für das Flüssigkeitsmanagement in einem persönlichen Hygieneprodukt.

Stand der Technik ist es separat hergestellte Deckblätter und Aufnahme-und Verteillagen während des Herstellvorgangs übereinander abzulegen und mittels Hotmeltverklebung oder thermischer Verschweißung miteinander zu verbinden.

**Gattungsbildend dazu sind die** US2005/0227563 A1**, wobei eine Lage aus einem Spinnvlies besteht, welches Filamente mit nicht-runden Querschnitten beinhaltet. Die** DE69230246 T2 **beschreibt ebenfalls die Kombination einer Aufnahmelage ("Vliesmaskierungsschicht") mit einer Verteillage ("Vliesverteilungsschicht"), wobei die Schichten geformte Fasern in unterschiedlichen Anteilen enthalten.**

Diese mehrlagigen Aufbauten haben den Nachteil der Grenzschichtenbildung am Übergang der Deckschicht zur nachfolgenden Aufnahme-und Verteilschicht. Durch den Hotmelt oder auch durch Tatsache, dass sich die Lagen bei thermischer Verschweißung nur bereichsweise miteinander verbunden sind, kommt es zu Beeinträchtigungen der Flüssigkeitsweiterleitung vom Deckblatt zur Aufnahme- und Verteilschicht. Dadurch bedingt steigen die Ansaugzeiten an.

Des Weiteren sind in den Verarbeitungsanlagen immer mehrere Abwickelstationen, sowie Verbindungseinheiten (Hotmelt oder Verschweißung) notwendig, damit die einzelnen Lagen mit einander verbunden werden können.

Aufgabe war es daher, ein Deckblatt zu schaffen, welches die Nachteile des Standes der Technik vermeidet. Gelöst wird die Aufgabe anhand der Merkmale des Anspruches 1, vorteilhafte Ausgestaltungen sind in den Ansprüchen 2 - **5** genannt.

Die nachstehenden Begriffe werden in der vorliegenden Erfindung genannt, wobei diese sich wie folgt definieren:
Bindefasern: bei den zum Einsatz kommenden Bindefasern handelt es sich um hydrophile Fasern, welche aus mehreren, thermoplastischen Komponenten bestehen. Diese Komponenten liegen als Side-by-Side Strukturen, als "Sea-Island" oder, erfindungsgemäß bevorzugt in Form einer Kern/Mantel-Struktur (zentrisch oder exzentrisch) vor. Kennzeichnend ist, dass eine Komponente einen Schmelzpunkt aufweist, der mindestens um 10°C höher liegt als der Schmelzpunkt einer ebenfalls in der Bindefaser vorhandenen zweiten Komponente. Zum Einsatz kommen bevorzugt Kern-/Mantel Fasern, die eine Kombination aus Polyethylen als niedrigschmelzende Komponente im Mantel und aus Polypropylen oder einem Polyester im Kern aufweisen. Derartige Fasern lassen sich problemlos mit den meist aus Polyethylen bestehenden Rückenblätter verschweißen. Denkbar sind aber auch andere Kombinationen mit bspw Polypropylen, Co-Polyester, Co-Polyamid, elastischen Polyestern oder anderen Thermoplasten als niedrigschmelzenden Mantel mit höherschmelzenden Thermoplasten wie beispielsweise Polyestern, elastischen Polyestern oder Polyamiden im Kern. Die Bindefasern haben dabei eine Feinheit im Bereich von 1,3dtex bis 6,7dtex, die Faserlänge richtet sich nach dem jeweils gewählten Herstellverfahren, sie liegt verfahrensbezogen zwischen 5 und 70mm

Füllfasern: es handelt sich dabei um homopolymer aufgebaute, hydrophile, synthetische Fasern, aus vorzugweise thermoplastischen Polymeren wie Polypropylen oder Polyester.
Die Füllfasern haben dabei eine Feinheit im Bereich von 1,0dtex bis 6,7dtex, die Faserlänge richtet sich nach dem jeweils gewählten Herstellverfahren, sie liegt verfahrensbezogen zwischen 5 und 60mm

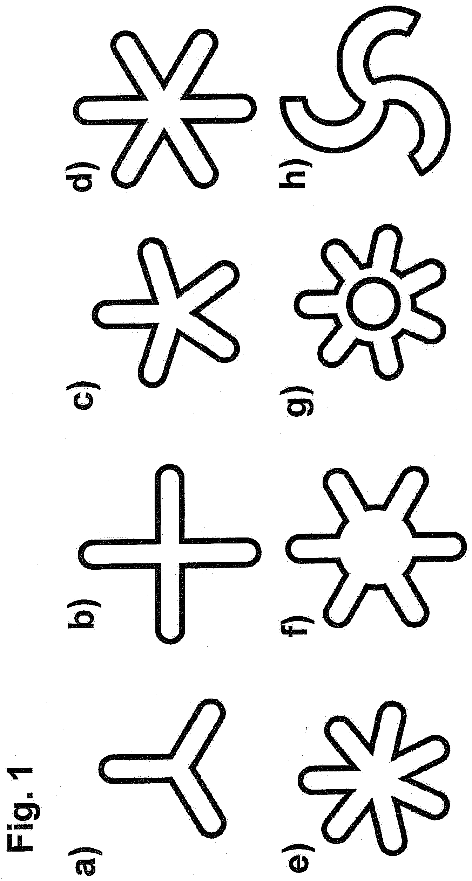

Geformte Fasern: es handelt sich dabei um homopolymer aufgebaute, synthetische, hydrophile Fasern, aus vorzugweise thermoplastischen Polymeren. Kennzeichnend für geformte Fasern im Sinne dieser Erfindung ist das Vorliegen eines nicht runden Faserquerschnittes, der eine Kapillarwirkung entlang der Faseroberfläche zur Folge hat. Bespiele für erfindungsgemäß geeignete Faserquerschnitte sind in Figur 1 gezeigt. Erfindungsgemäß werden gemäß der Form a) aus Figur 1 geformte Fasern bevorzugt, es können aber quadrolobal (vergleiche erfindungsgemäßes Deckblatt 4), pentalobal oder auch unsymetrische, multilobale Querschnitte zum Einsatz kommen. Die Verteilfasern haben dabei eine Feinheit im Bereich von 1,0dtex bis 6,7dtex, die Faserlänge richtet sich nach dem jeweils gewählten Herstellverfahren, sie liegt verfahrensbezogen zwischen 5 und 60mm

Trockenverfahren: bezeichnet das Herstellverfahren für erfindungsgemäße Deckblätter. Erfindungsgemäße Deckblätter können nach einem im Buch "Vliesstoffe", erschienen im Verlag Wiley VCH, 2. Auflage, 2012 aufgezeigten Trockenverfahren hergestellt werden. Erfindungsgemäß bevorzugt, aber ohne darauf beschränkt zu sein, wird das Kardierverfahren unter Verwendung von Stapelfasern.

Heißluftverfestigung: bezeichnet ein Verfestigungsverfahren, welches bei der Herstellung der erfindungsgemäßen Deckblätter zur Anwendung kommt. Die grundlegenden Techniken dazu werden im Buch "Vliesstoffe", erschienen im Verlag Wiley VCH, 2. Auflage, 2012, Seiten 375-382 beschrieben. Erfindungsgemäß bevorzugt, aber ohne darauf beschränkt zu sein, wird ein Verfahren zur Heißluftverfestigung unter Verwendung eines Band- oder Trommeltrockners.

Kalanderverfestigung: bezeichnet ein Verfestigungsverfahren, welches gegebenenfalls an die Heißluftverfestigung für erfindungsgemäße Deckblätter anschließt. Die grundlegenden Techniken dazu werden im Buch "Vliesstoffe", erschienen im Verlag Wiley VCH, 2. Auflage, 2012, Seiten 385-395 beschrieben. Erfindungsgemäß bevorzugt, aber ohne darauf beschränkt zu sein, wird ein Thermobond-Kalander, d.h. ein mittels Druck- und Hitze arbeitendes System. Dabei sind strukturgegebende Kalander wie beispielsweise ineinandergreifende Male/Female Walzen einsetzbar, erfindungsgemäß bevorzugt wird jedoch eine Kombination aus glatter Walze und gravierter Gegenwalze, was eine punktweise Verfestigung zur Folge hat. Die Verwendung eines Ultraschallkalanders ist ebenso möglich.

Für die in Tabelle 1 genannten Prüfparameter wurden die nachfolgend genannten Prüfmethoden verwendet:
- Flächengewicht: gemäß WSP 130.1, Angabe in g/m²
- Dicke: gemäß WSP 120.6, ermittelt bei 0,02kPa Messdruck, Angabe in mm
- Ansaugzeit 1: gemäß WSP 70.7, Angabe in s
- Ansaugzeit 2 und 3: in Anlehnung an WSP 70.7. Aufbau wie unter Ansaugzeit 1 beschrieben, jedoch wird das Materialmuster nach Ermittlung der Ansaugzeit 1 noch zweimal im Abstand von jeweils 60 Sekunden mit 5ml Testflüssigkeit an der gleichen Stelle erneut beaufschlagt. Das darunterliegende Saugpapier wird nicht gewechselt.
- Rücknässung: gemäß WSP 70.8, Angabe in g
- Maximalkraft Längs / Quer: gemäß WSP 110.4, Opt.B, Angabe in N/50mm

Die Ansaugzeit 1 beschreibt die Eigenschaft eines Deckblatts, Flüssigkeiten schnell aufzunehmen. Die Ansaugzeiten 2 und 3 beschreiben das Verhalten eines Aufnahme- und Verteilvliesstoffes, die vom Deckblatt weitergeleitete Flüssigkeitsmenge vom ursprünglichen Auftreffpunkt wegzuleiten über die Breite und Länge der Lage zu verteilen und an den Saugkörper zu übergeben.

Der bisherige Stand der Technik geht wie eingangs beschrieben von einer Kombination von Deckblatt und Verteillage aus. Das dem Körper zugewandte Deckblatt besteht aus Vliesstoff, welcher, um einen weichen Griff zu erzeugen, aus Fasern mit geringen Faserdurchmessern im Bereich von 1,0 dtex bis 2,2 dtex besteht. Eingesetzt werden häufig Polypropylen-Spunbond-Vliese als Polymer für diese Deckblätter. Das Flächengewicht derartiger Lagen liegt zwischen 10 und 20 g/m².

Diesem Deckblatt nach dem Stand der Technik folgt eine Verteillage aus Fasern mit höheren Faserdurchmessern, d.h. im Bereich von 2,2 bis 6,7 dtex, zum Teil sogar bis 10dtex. Diese Lagen bewirken die Verteilung der vom Deckblatt angesaugten Flüssigkeit, sodass ein Maximum der der Verteilschicht folgenden Speicherschicht langsam mit Flüssigkeit beladen wird und so dem Gel-Blocking bzw der Rücknässung vorgebeugt wird. Eine dem Stand der Technik entsprechende Verteillage hat mechanisch geringe Beständigkeiten, welches dem Vorhandensein von nur wenigen Verbindungsstellen innerhalb der Lage geschuldet ist. Da solche Lagen entweder an das Deckblatt oder an die Speicherschicht gebunden sind, ist dies im Allgemeinen auch unkritisch.

Im Unterschied zum bekannten Stand der Technik vereint ein erfindungsgemäß ausgeführtes Deckblatt nun die Flüssigkeitsansaug-Eigenschaften eines bekannten Deckblatts und die Speicher- und Verteileigenschaften einer bekannten Verteillage innerhalb einer Lage.

Mittels einer erfindungsgemäß ausgeführten Deckblatt-Lage sind die gleichen technischen Werte bezüglich Ansaugung, Verteilung und Rücknässung zu erzielen, die bisher nur durch die Kombination mindestens zweier Lagen möglich waren.

Bei der Herstellung eines erfindungsgemäß ausgeführten Deckblatts kommen hydrophile Fasermischungen zum Einsatz, die aus Bindefasern, Verteilfasern und Stützfasern bestehen.

Die Bindefasern gewährleisten dabei, nachdem sie im Rahmen der Verfestigung aktiviert wurden, die mechanische Stabilität des resultierenden, erfindungsgemäßen Deckblatts. Sie haben auch Einfluss auf die Haptik, da Schmelzfasern mit olefinischen Mantelpolymeren (PP oder PE) einen seifigen Griff zur Folge haben, der vom Benutzer als weich wahrgenommen wird. Des Weiteren bestehen die in persönlichen Hygieneprodukten verwendeten Rückenblätter bestehen zumeist aus Polyethylen. Bindefasern im Deckblatt mit Polypropylen oder Polyethylen als Mantelpolymer lassen sich hier leichter mit dem Rückenblatt verschweißen und werden daher bevorzugt eingesetzt. Für spezielle Anwendungen kommen auch elastische Bindefasern, wie beispielsweise in der WO9119032 offenbart, zum Einsatz.

Die geformten Fasern haben aufgrund ihres nicht-runden Faserquerschnitts eine deutlich vergrößerte Faseroberfläche entlang derer auftreffende Flüssigkeit aufgrund der Kapillarwirkung an nachfolgende Speicherschichten weitergeleitet wird. Faseravivagen können diese Kapillarwirkung verstärken. Mögliche Faserquerschnitte sind in Figur 1 beispielhaft und ohne darauf beschränkt zu sein, gezeigt. Bevorzugt verwendet werden trilobal (Form a)) oder quadrolobal (Form b)) ausgeformte Faserquerschnitte.

Die Stützfasern haben, sofern in der Mischung enthalten, die Aufgabe, dem Deckblatt weiteren Bausch zu verleihen und eine kostengünstigere Herstellung zu ermöglichen. Die Stützfasern sind für das Flüssigkeitsmanagement nicht unbedingt notwendig, daher kann ein erfindungsgemäßes Deckblatt auch ohne Stützfasern ausgeführt sein. Sie werden dann verwendet, wenn das Deckblatt eine Abstandshalterfunktion ausüben soll und besondere hohe Druckbelastungen im Gebrauch auftreten, wie dies beispielsweise bei Erwachseneninkontinenz-Einlagen der Fall ist. Anteile können bis maximal 30 Gewichtsprozent liegen.

Die Verfestigung des erfindungsgemäßen Deckblatts erfolgt primär thermisch mittels des Heißluft-Verfahrens, wobei sich diesem Verfestigungsschritt eine weitere Kalanderverfestigung anschließen kann.

Durch die Kalanderverfestigung wird die Dichte eines erfindungsgemäßen Deckblatts erhöht. Es bilden sich dabei vermehrt stellen aus, an welchen sich die geformten Fasern gegenseitig berühren. Dadurch erhöht sich die potenziell zur Flüssigkeitsweiterleitung vorhandene Faseroberfläche, was insgesamt zu einer Vergleichmäßigung der Flüssigkeitsleitung führt. Des Weiteren ergibt durch die Kalanderbehandlung eine Hoch/Tief-Struktur auf der Oberfläche des Deckblatts. Diese wirkt sich positiv auf das Flüssigkeithandling, insbesondere bei niederviskosen Flüssigkeiten aus.

Die Notwendigkeit einer Kalanderbehandlung ist des Weiteren abhängig von der für den Einsatzzweck benötigten Maximalkraft des Deckblatts und von der Notwendigkeit, das Abflusen der geformten Fasern und/oder die Stützfasern zu verhindern.

Sollte die Kalanderverfestigung benötigt werden, so sind Verfestigungsflächenanteile von kleiner 20%, bevorzugt unter 12%, und besonders bevorzugt kleiner 8% zu verwenden.

Der Anteil an Bindefasern in einem erfindungsgemäß ausgeführten Deckblatt liegt zwischen 25 und 75 Gewichtsprozent. Der Anteil ist von der Maximalkraft, von der Verbindung zur nachfolgenden Speicherschicht und von der Haptik des Deckblatts abhängig. Ist ein weicher, seifiger Griff gewünscht, sind Anteile zwischen 40 und 75 Gewichtsprozent zu verwenden. Weist das Deckblatt viele Verbindungspunkte, bspw. Verschweißungen zu nachfolgenden Schichten auf, werden Anteile von 25 bis 40 Gewichtsprozent eingesetzt.

Der Anteil an geformten Fasern richtet sich nach den Flüssigkeitsleit- und Verteileigenschaften. Größere Flüssigkeitsmengen und weite Verteilung der Flüssigkeit über die Speicherschicht verlangen Anteile von 40 bis 50 Gewichtsprozent. Sind die auftreffenden Flüssigkeitsmengen eher gering kann mit Anteilen zwischen 25 und 40 Gewichtsprozent gearbeitet werden.

Anzustreben sind Flächengewichte im Bereich von 15 bis 25 g/m², wobei dies jeweils dem Anforderungsprofil anzupassen. Die genannten Ausführungsbeispiele wurden aus Gründen der Vergleichbarkeit alle in einem Flächengewicht von 25g/m² ausgeführt.

Die Tabelle 1 zeigt einen Vergleich von erfindungsgemäß ausgeführten Deckblättern 3 bis **8** mit Materialien bzw Materialaufbauten **1, 2 und 9** die dem Stand der Technik entsprechen **bzw als Vergleichsbeispiel dienen.**

Dabei sind die einzelnen Materialien wie folgt aufgebaut bzw hergestellt:
Im Vergleichsbeispiel 1 und den erfindungsgemäß ausgeführten Deckblättern 3, 6 und 7 wurden als Bindefasern hydrophile Polyethylen / Polypropylen Bikomponenten-Fasern, im Weiteren als "PE/PP-Fasern" bezeichnet, in einem Titer von 1,7dtex, Stapellänge 40mm, erhältlich von FiberVisions, Dänemark mit der Handelsbezeichnung "ES-C 215" verwendet.

In den erfindungsgemäß ausgeführten Deckblättern 4,5, **und 8** kommen hydrophile Polyethylen- Polyester Bikomponenten-Fasern, im Weiteren als "PE/PET-Fasern" bezeichnet, in einem Titer von 2,2dtex, Stapellänge 40mm, erhältlich von FiberVisions, Dänemark mit der Handelsbezeichnung "ETC-Bounce" zum Einsatz.

Die geformten Fasern in den erfindungsgemäß ausgeführten Deckblättern 3, sowie 5-**8** sind trilobal geformte Polypropylenfasern in einem Titer von 2,2dtex, Stapellänge 40mm, erhältlich von FiberVisions, Dänemark mit der Handelsbezeichnung "High-Loft".

Im erfindungsgemäß ausgeführten Deckblatt 4 werden als geformte Fasern quadrolobal geformte Polyesterfasern 1,5dtex ,38mm des Herstellers FarEastern, Taiwan, Type Eastlon SN-34381 WXX verwendet.

Die Füllfasern in den erfindungsgemäß ausgeführten Deckblättern **5 und 8** sind normale Polyesterfasern mit rundem Querschnitt mit einem Titer von 1,3dtex, 40mm des Herstellers Trevira, Deutschland mit dem Handelsnamen Type 298.

Beim Deckblatt 1 handelt es sich um ein dem Stand der Technik entsprechendes kardiertes Vlies mit einem Flächengewicht von 25g/m², bestehend aus 100% PE/PP-Fasern. Das Vlies wurde im Heißluftverfahren verfestigt. Die Ermittlung der Ansaugzeiten fand ohne die Kombination mit einer Aufnahme- und Verteillage statt.

Beim Deckblatt 2 wurde eine handelsübliches, hydrophiles 15g/m² Spunbond Deckblatt aus Polypropylenfilamenten im Titer von 1,7dtex mit einer dem Stand der Technik entsprechenden Aufnahme- und Verteillage kombiniert (Bezeichnung ADL). Die Aufnahme- und Verteillage besteht dabei aus einem wasserstrahlverfestigten Stapelfaservliesstoff im Gewicht von 50g/m² und einer Mischung aus 22,5% einer Viskosefaser im Titer von 1,7dtex/40mm mit 77,5% einer bikomponenten Bindefaser aus Polyethylen im Mantel und Polethylenterephthalat im Kern mit einem Titer von 4,4dtex. Die Aufnahme- und Verteillage ist erhältlich bei der Sandler AG, Deutschland mit der Bezeichnung sawasoft 2674. Das Flächengewicht des kombinierten Produktes liegt bei 65g/m².

Das erfindungsgemäß ausgeführte Deckblatt 3 besteht aus einer Mischung von 50 Gewichtsprozent der PE/PP-Faser mit 50 Gewichtsprozent einer trilobal geformten PP-Faser. Die Fasern wurden zunächst gemischt und dann einer Kardieranlage zugeführt. Der so hergestellte Faserflor wurde dann mit einem Trommeltrockner mit 270° Umschlingung einer Heißluft-Verfestigung während 3 Sekunden bei einer Temperatur von 132°C unterzogen. Das sich ergebende Flächengewicht liegt bei 25g/m²

Das erfindungsgemäß ausgeführte Deckblatt 4 besteht aus einer Mischung von 75 Gewichtsprozent PE/PET-Faser mit 25 Gewichtsprozent einer quadrolobal geformten PET-Faser. Die Fasermischung wurde zunächst gemischt und dann einer Kardieranlage zugeführt. Der so hergestellte Faserflor wurde dann mit einem Trommeltrockner mit 270° Umschlingung einer Heißluft-Verfestigung während 5 Sekunden bei einer Temperatur von 132°C unterzogen. Das sich ergebende Flächengewicht liegt bei 25g/m²

Das erfindungsgemäß ausgeführte Deckblatt 5 besteht aus einer Mischung von 50 Gewichtsprozent PE/PET-Faser (Kern-Mantel-Struktur), 25 Gewichtsprozent einer trilobal geformten PP-Faser und 25 Gewichtsprozent einer Füllfaser. Die Fasermischung wurde zunächst gemischt und dann einer Kardieranlage zugeführt. Der so hergestellte Faserflor wurde dann mit einem Trommeltrockner mit 270° Umschlingung einer Heißluft-Verfestigung während 5 Sekunden bei einer Temperatur von 132°C unterzogen. Das sich ergebende Flächengewicht liegt bei 25g/m²

Das erfindungsgemäß ausgeführte Deckblatt 6 besteht aus dem im Vergleichsbeispiel 3 hergestellten, erfindungsgemäßen Deckblatt. Im Anschluss an die Heißluftverfestigung wurde eine weitere Kalanderbehandlung bei einer Temperatur von 125°C durchgeführt. Dabei betrug die Verfestigungsfläche 5% bei 9 Figuren pro cm², die Geschwindigkeit bei der Kalanderpassage lag bei 5 m/min, der Druck bei 25daN/cm.

Das erfindungsgemäß ausgeführte Deckblatt 7 besteht aus dem im Vergleichsbeispiel 3 hergestellten, erfindungsgemäßen Deckblatt. Im Anschluss an die Heißluftverfestigung wurde eine weitere Kalanderbehandlung bei einer Temperatur von 125°C durchgeführt. Dabei betrug die Verfestigungsfläche 20% bei 11 Figuren pro cm², die Geschwindigkeit bei der Kalanderpassage lag bei 5 m/min, der Druck bei 50daN/cm.

Das erfindungsgemäß ausgeführte Deckblatt 8 besteht aus dem im Vergleichsbeispiel 5 hergestellten, erfindungsgemäßen Deckblatt. Im Anschluss an die Heißluftverfestigung wurde eine weitere Kalanderbehandlung bei einer Temperatur von 125°C durchgeführt. Dabei betrug die Verfestigungsfläche 5% bei 9 Figuren pro cm², die Geschwindigkeit bei der Kalanderpassage lag bei 5 m/min, der Druck bei 25daN/cm.

Das **Vergleichsbeispiel** Deckblatt 9 besteht aus **der** im **Deckblatt** 5 **verwendeten Fasermischung. Im** Anschluss an die Heißluftverfestigung wurde eine weitere Kalanderbehandlung bei einer Temperatur von 125°C durchgeführt. Dabei betrug die Verfestigungsfläche 20% bei 11 Figuren pro cm², die Geschwindigkeit bei der Kalanderpassage lag bei 5 m/min, der Druck bei 50daN/cm.

In der Tabelle 1 werden als Kennzeichen für die gewählte Verfestigungsart folgende Kürzel verwendet:
HL = Heißluftverfestigung,
HL/K = Heißluftverfestigung + Kalanderverfestigung
5/9: Kalanderverfestigung mit einer Pressfläche von 5% bei 9 elliptisch geformten Pressflächen pro cm²
20/11: Kalanderverfestigung mit einer Pressfläche von 20% bei 11 elliptisch geformten Pressflächen pro cm²

Betrachtet man die Tabelle 1 so kann man folgendes erkennen:
Die dem Stand der Technik entsprechenden Deckblätter 1 und 2 haben im Vergleich zu den erfindungsgemäß ausgeführten Deckblättern 3 bis **8** deutlich höhere Festigkeiten. Dies ist jedoch für den Einsatzzweck im Bereich der Damenhygiene unerheblich, da die Festigkeit eines Deckblatts aufgrund der bei der Verarbeitung eingebrachten Verbindungsstellen mit dem Rückenblatt bzw der Speicherlage nicht von entscheidender Bedeutung ist.

Die erfindungsgemäß ausgeführten Deckblätter 3 bis 8 eine Rücknässung aufweisen, die dem des Standes der Technik entsprechen.

Die Deckblätter 1 und 2 haben des Weiteren im Vergleich zu den erfindungsgemäß ausgeführten Deckblättern deutlich höhere Ansaugzeiten 1. Dies ist beim Deckblatt 1 durch die hohe Anzahl von lagen-internen Verbindungsstellen der bikomponenten Fasern untereinander verursacht. Bevor Flüssigkeit weitergeleitet wird, bilden sich, bedingt durch die Oberflächenspannung der Flüssigkeit, zunächst an Faser-Faserverbindungssstellen Spannsegel-artige Flüssigkeitsansammlungen, die erst verzögert an das Saugpapier weitergeleitet werden. Die Faser-Faserverbindungsstellen wirken als Störstellen beim Flüssigkeitstransport und behindern diesen.

Beim Deckblatt 2 ist durch die Grenzschicht am Übergang zur Aufnahme-und Verteillage die Ansaugzeit 1 geringer, steigt aber dennoch bei der zweiten und dritten Flüssigkeitsbeaufschlagung an.

**Beim Vergleichsbeispiel Deckblatt 9 ist durch die Einbringung von 25% Füllfasern in Verbindung mit einer Kalanderverfestigungsfläche von 20% die Ansaugzeit 3 höher im Vergleich zu dem erfindungsgemäß ausgeführten Deckblatt 7.**

Das erfindungsgemäß ausgeführte Deckblatt 3, bestehend aus einer Mischung von 50% Bindefasern mit 50% trilobal geformten Fasern, zeigt hier eine deutlich verkürzte Ansaugzeit 1, der Einfluss der geformten Fasern und das Fehlen von Zonenübergängen ist deutlich zu erkennen. Die Flüssigkeitsableitung geschieht entlang der Faseroberfläche der geformten Fasern, sodass die auftreffende Flüssigkeit störstellenfrei an das gemäß der Testprozedur nachfolgende Saugpapier weitergegeben wird. Durch die im Vergleich zum Deckblatt 1 verminderte Anzahl von Störstellen im Faserverbund lassen dabei auch bei den Ansaugzeiten 2 und 3 deutlich geringere Werte im Vergleich zum Stand der Technik erzielen. Dies ist dann vorteilhaft, sollte im späteren Gebrauch mehrmalige Flüssigkeitsbeaufschlagung erwartet werden (bspw bei Inkontinenzprodukten).

Eine Verringerung des Anteils an geformten Fasern wie im Deckblatt 4 gezeigt, wirkt sich in einer höheren Ansaugzeit 1 aus, wobei die Ansaugzeit 1 immer noch deutlich unter der des Deckblatts 1 liegt. Man kann aber bereits den Einfluss der höheren Anzahl von Störstellen erkennen, die sich aufgrund des Anteils von 75 Gewichtsprozent Bindefasern ergibt. Der Anteil an Bindefasern sollte daher den Wert von 75 Gewichtsprozent nicht übersteigen, da sich dann die Ansaugzeit 1 weiter erhöht und sich immer mehr dem Stand der Technik angleicht.

Das ebenfalls erfindungsgemäß ausgeführte Deckblatt 5 enthält neben 50 Gewichtsprozent Bindefasern und 25 Gewichtsprozent geformten Fasern auch einen Anteil von 25 Gewichtsprozent Polyester-Füllfasern. Die Verwendung von Füllfasern, welche im Vergleich zu den geformten Fasern wesentlich kostengünstiger sind, bietet sich dann an, wenn im späteren Gebrauch nur wenig Flüssigkeitsbeaufschlagung erwartet wird. Wie man der Tabelle 1 entnehmen kann, ist das Ergebnis für die Ansaugzeit 1 des Deckblatts 5 der des Deckblatts 3 vergleichbar. Unterschiede ergeben sich erst bei vermehrter Beaufschlagung, in Tabelle bei den Ansaugzeiten 2 und 3, die dann leicht ansteigen.

Die Dicke des Deckblattes 5 ist dabei mit 2,22mm vergleichbar zu dem Deckblatt 2, einem dem Stand der Technik entsprechenden und zweischichtig aufgebauten Material, wobei der Vorteil des erfindungsgemäß ausgeführten Deckblatts 5 die Einlagigkeit ist.

Betrachtet man die erfindungsgemäß ausgeführten Deckblätter 6 bis **8** so erkennt man, dass eine an die Heißluftverfestigung anschließende Kalanderverfestigung der beschriebenen Art erwartungsgemäß zu einer Verringerung der Dicke führt. Überraschend wurde jedoch festgestellt, dass die Kalanderverfestigung einen positiven Einfluss auf die Entwicklung der Ansaugzeiten 2 und 3, d.h. bei mehrmaliger Flüssigkeitsbeaufschlagung hat. Begründet ist dies durch eine Erhöhung der Dichte und damit der Kapillarität eines derart kombiniert verfestigten Deckblatts. Durch die der Heißluftverfestigung folgenden Kalanderpassage wird das Vlies verdichtet und dieser verdichtete Zustand durch die Verschweißungen an den Printpunkten partiell fixiert. Des Weiteren führt die eingeprägte Hoch / Tief-Struktur wie vorerwähnt zu einem besseren Flüssigkeitshandling, insbesondere auch bei niederviskosen Flüssigkeiten.

Die Deckblätter 6 und 8 wurden mit einem Kalander, bestehend aus einer glatten Walze, auf die eine gravierte Walze drückt, verfestigt. Die gravierte Kalanderwalze weist dabei eine Verfestigungsfläche von 5% bei einem Anteil von 9 Figuren pro cm² auf. Die Printpunkte sind ellipsenförmig ausgeführt.

Die Kalanderverfestigung führt zu einer Vergleichmäßigung der Ansaugzeit, sodass die Ergebnisse für die Ansaugzeiten 1, 2 und 3 annähernd gleich gering sind.

Wählt man die Verfestigungsfläche der gravierten Walze höher, wie **im Deckblatt** 7 auf den Wert von 20%, so ergeben sich für die Ansaugzeiten 2 und 3 Werte, die sich wieder denen der Ausgangsmaterialien, das heißt dem Stand der Technik entsprechenden Materialkombinationen angleichen. Eine Verfestigungsfläche von 20% wird daher als Obergrenze angesehen.

Zusammenfassend ergibt sich, dass die erfindungsgemäß beanspruchte Fasermischung bestehend aus geformten Fasern mit Bindefasern und ggf Füllfasern, welche nach der Flächenbildung mittels Heißluftverfestigung oder, erfindungsgemäß bevorzugt, mittels einer Kombination aus Heißluftverfestigung und Kalanderverfestigung verfestigt wird, Deckblätter ergibt, die dem Stand der Technik deutlich überlegen sind.

Bei im Vergleich zum Stand der Technik gleicher oder verbesserter Produktleistung bewirken erfindungsgemäß hergestellte Deckblätter
a) die Vereinigung der Eigenschaften eines Deckblatts mit den Eigenschaften einer Aufnahme-und Verteillage in einer Materiallage
b) die einfachere Weiterverarbeitung, da aufgrund der Einlagigkeit der Aufwand an der Verarbeitungsmaschine reduziert wird.
c) die einfache Anpassung der Flüssigkeits-Handlingseigenschaften und Kostenforderungen durch Verschiebung der Mischungsverhältnisse der Einzelkomponenten zu einander
d) eine Materialersparnis im Fertigprodukt.
e) eine Schonung der Ressourcen aufgrund geringerem Rohmaterialeinsatz

**Tabelle 1:**

| Deckblatt | 1 | 2 | 3 | 4 | 5 | 6 | 7 | 8 | 9 |
|---|---|---|---|---|---|---|---|---|---|
| Materialzusammensetztung | 100% PE/PP - Bindefaser | PP-Spunbond + ADL | 50% PE/PP-Bindefaser + 50% PP Trilobal | 75% PE/PET - Bindefaser + 25% PET Quadrolobal | 50% PE/PET-Bindefaser + 25% PP Trilobal + 25% PET Füllfaser | 50% PE/PP-Bindefaser + 50% PP Trilobal | 50% PE/PP-Bindefaser + 50% PP Trilobal | 50% PE/PET-Bindefaser + 25% PP Trilobal + 25% PET Füllfaser | 50% PE/PET-Bindefaser + 25% PP Trilobal + 25% PET Füllfaser |
| Flächengewicht (g/m²) | 25 | 65 | 25 | 25 | 25 | 25 | 25 | 25 | 25 |
| Verfestigung | HL | HL | HL | HL | HL | HL/K 5/9 | HL/K 20/11 | HL/K 5/9 | HL/K 20/11 |
| Maximalkraft Längs (N/50mm) | 44,6 | 25,1 | 12,9 | 9,5 | 4,3 | 9,8 | 20,7 | 5,7 | 11,3 |
| Maximalkraft Quer (N/50mm) | 24,7 | 13,2 | 4,4 | 3,8 | 1,4 | 1,5 | 5,3 | 1,9 | 2,5 |
| Ansaugzeit 1 (s) | 5,47 | 1,70 | 0,59 | 1,03 | 0,53 | 1,13 | 0,58 | 1,95 | 1,02 |
| Ansaugzeit 2 (s) | 2,73 | 2,85 | 1,98 | 1,80 | 1,68 | 1,45 | 1,83 | 1,95 | 2,63 |
| Ansaugzeit 3 (s) | 2,98 | 4,40 | 1,88 | 3,35 | 2,35 | 1,71 | 3,20 | 1,68 | 5,27 |
| Rücknässung (g) | 0,10 | 0,11 | 0,11 | 0,11 | 0,10 | 0,10 | 0,10 | 0,10 | 0,10 |
| Dicke bei 0,02kPa Messdruck (mm) | 1,13 | 1,90 | 1,58 | 2,38 | 2,22 | 1,15 | 0,98 | 1,19 | 1,02 |

## Patentansprüche

1. Thermisch verfestigtes Deckblatt für persönliche Hygieneartikel, bestehend aus thermoplastischen **Stapel**-Fasermaterialien
**dadurch gekennzeichnet, dass**
- das Deckblatt
o einen Gehalt von 0 bis 30 Gewichtsprozent einer Füllfaser,
o einen Gehalt von 25 bis 75 Gewichtsprozent einer Bindefaser und
o einen Gehalt von 25 bis 50 Gewichtsprozent einer synthetischen homopolymeren, hydrophilen Faser, welche einen geformten, nicht runden Querschnitt hat aufweist,
- die das Deckblatt bildenden Faserstoffe homogen miteinander vermischt sind.
- das Deckblatt eine Ansaugzeit 1 von kleiner 2 Sekunden, eine Ansaugzeit 2 von kleiner 3 Sekunden und eine Ansaugzeit 3 von kleiner 4 Sekunden aufweist, wobei die Ansaugzeiten gemäß der in der Beschreibung genannten Methode ermittelt werden und
- das Deckblatt einlagig ist.

2. Deckblatt gemäß Anspruch 1
**dadurch gekennzeichnet, dass**
- das Deckblatt thermisch mittels Heißluft gebunden ist.

3. Deckblatt gemäß Anspruch 2
**dadurch gekennzeichnet, dass**
- das Deckblatt zusätzlich thermisch mittels Kalandrierung gebunden ist.

4. Deckblatt nach einem der vorgehenden Ansprüche
**dadurch gekennzeichnet, dass**
das Deckblatt ein Flächengewicht von 15 bis 50 g/m² aufweist.

5. Persönlicher Hygieneartikel,
**dadurch gekennzeichnet, dass**
ein Deckblatt nach einem der vorhergehenden Ansprüche enthalten ist.

## Claims

1. A thermally solidified cover sheet for personal hygiene items, consisting of thermoplastic stacked fibre materials
**characterized in that**
- the cover sheet has
∘ a fibrefill content of 0 to 30 weight percent,
∘ a binding fibre content of 25 to 75 weight percent and
∘ content of 25 to 50 weight percent of a synthetic homopolymeric, hydrophilic fiber having a shaped, non-round cross section,
- the fibrous materials which bind the cover sheet are homogeneously mixed together,
- the cover sheet has a suction time 1 of less than 2 seconds, a suction time 2 of less than 3 seconds and a suction time 3 of less than 4 seconds, wherein the suction times are determined according to the method cited in the description, and
- the cover sheet is a single layer.

2. The cover sheet according to claim 1,
**characterized in that**
- the cover sheet is thermally bonded using hot air.

3. The cover sheet according to claim 2,
**characterized in that**
- the cover sheet is also thermally bonded by means of calendering.

4. The cover sheet according to one of the preceding claims,
**characterized in that**
- the cover sheet has a grammage of 15 to 50 g/m².

5. A personal hygiene item,
**characterized in that**
- a cover sheet according to one of the preceding claims is contained therein.

## Revendications

1. Feuille de couverture fixée par voie thermique pour des articles d'hygiène personnelle, constituée de matériaux fibreux thermoplastiques empilés, **caractérisée en ce que**
- la feuille de couverture présente
∘ une teneur de 0 à 30 pour cent en poids d'une fibre de charge,
∘ une teneur de 25 à 75 pour cent en poids d'une fibre de liaison, et
∘ une teneur de 25 à 50 pour cent en poids d'une fibre synthétique homopolymère, hydrophile, laquelle a une section transversale de forme non circulaire,
- les matériaux fibreux formant la feuille de couverture sont mélangés de manière homogène,
- la feuille de couverture a un temps d'absorption 1 inférieur à 2 secondes, un temps d'absorption 2 inférieur à 3 secondes et un temps d'absorption 3 inférieur à 4 secondes, les temps d'absorption étant déterminés selon le procédé cité dans la description, et
- la feuille de couverture est monocouche.

2. Feuille de couverture selon la revendication 1,
**caractérisée en ce que**
- la feuille de couverture est liée par voie thermique au moyen d'air chaud.

3. Feuille de couverture selon la revendication 2,
**caractérisée en ce que**
- la feuille de couverture est en outre liée par voie thermique au moyen d'un calandrage.

4. Feuille de couverture selon l'une des revendications précédentes,
**caractérisée en ce que**
- la feuille de couverture présente un poids surfacique de 15 à 50 g/m².

5. Article d'hygiène personnel
**caractérisé en ce**
- **qu'**une feuille de couverture selon l'une des revendications précédentes y est contenue.
